# EUROPEAN PATENT APPLICATION

(11) **EP 4 098 655 A1**
(43) Date of publication of application: **07.12.2022**
(21) Application number: 20917061.2
(22) Date of filing: 14.12.2020
(51) Int. Cl.: C07H 21/02, C12N 15/10

(54) **METHOD FOR PRODUCING NUCLEIC ACID OLIGOMER**

(30) Priority: 29.01.2020 JP 2020012787
(71) Applicant: SUMITOMO CHEMICAL COMPANY, LIMITED, Tokyo 103-6020 (JP)
(72) Inventor: MIYAGAWA, Takuya, Osaka-shi, Osaka 555-0021 (JP); NISHIO, Masashi, Osaka-shi, Osaka 554-8558 (JP)
(74) Representative: Vossius & Partner Patentanwälte Rechtsanwälte mbB
(86) International application number: PCT/JP2020/046572
(87) International publication number: WO 2021/153047

(57) **Abstract**

The present invention provides an efficient process for preparing a nucleic acid oligomer, that is, a process for preparing a nucleic acid having a phosphate triester bond effectively by oxidizing a nucleic acid precursor having a phosphite triester bond. Also the present invention provides a process for preparing a nucleic acid compound having at its 5'-terminus a nucleotide represented by formula (I) by a phosphoramidite method, which comprises a step of reacting a precursor having a phosphite triester bond represented by a formula (II) (the definitions of substituents of formulae (I) and (II) are described in the Description) with an oxidation solution which contains iodine, pyridine and water, and a molar ratio of iodic acid to iodine (mole of iodic acid / mole of iodine) is 30 × 10⁻³ or less.

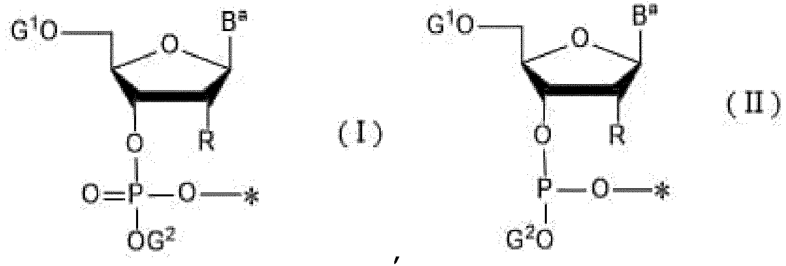

## Description

### TECHNICAL FIELD

This application claims priority to and the benefit of Japanese Patent Application No. 2020-012787 filed January 29, 2020, the entire contents of which are incorporated herein by reference.

The present invention relates to a process for preparing nucleic acid oligomer.

DNA and RNA which are a nucleic acid oligomer can be applied as a DNA probe, a RNA probe, an antisense, a ribozyme, a siRNA, or an aptamer and so on, which is a useful material.

A nucleic acid oligomer can be synthesized by a solid phase synthesis, and in the solid phase synthesis, a phosphoramidite of a nucleoside (hereinafter, referred to as "amidite") is used as a raw material. A nucleic acid oligomer that is synthesized by elongating a nucleic acid through a coupling step, an oxidation step and a deprotection step on a solid support is cut off from a solid support, and a protecting group is then removed to prepare a desired nucleic acid oligomer. However, the purity of the nucleic acid oligomer synthesized according to this method was not necessarily satisfactory, and the synthesis method was not thus efficient (see Non-Patent Literature 1).

### CITATION LIST

### NON-PATENT LITERATURE

No-Patent Literature 1: Tetrahedron 69 (2013) 3615-3637

### SUMMARY OF THE INVENTION

### (PROBLEMS TO BE SOLVED BY INVENTION)

An object of the present invention is to provide an efficient process for preparing a nucleic acid oligomer.

### (MEANS TO SOLVE PROBLEMS)

The present inventors have intensively studied to achieve the above object, and as a result, can find out an efficient process for preparing nucleic acid oligomer, which comprises in a synthesis of nucleic acid oligomer, as an oxidation solution to be used in an oxidation of phosphite ester which is produced by a coupling reaction using a phosphoramidite, using a solution which contains iodine, water and pyridine, and a ratio of iodic acid to iodine is below a certain level thereof.

The present invention encompasses the following aspects, but are not limited thereto.
[1] A process for preparing a nucleic acid compound having at its 5'-terminus a nucleotide represented by formula (I): 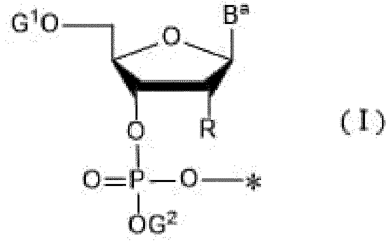 (wherein
   G¹ and G² each represents independently of each other a protecting group for hydroxy group, and B^{a} represents a nucleic acid base which may be optionally protected with a protecting group,
   R represents a protected hydroxy group, a hydrogen atom, a fluorine atom, a methoxy group, a 2-methoxyethyl group, or a OQ' group,
   Q' represents a methylene group attached to a carbon atom at 4'-position of ribose, an ethylene group attached to a carbon atom at 4'-position of ribose, or an ethylidene group attached to a carbon atom at 4'-position of ribose, and
   a bond marked with symbol * represents a bond directing to 3' terminus side of a nucleic acid) by a phosphoramidite method,
   which comprises a step of reacting a precursor having a phosphite triester bond represented by a formula (II): 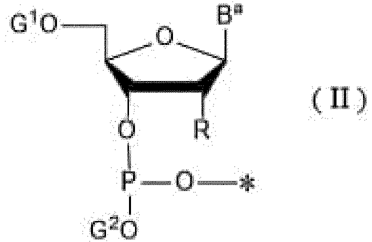 (wherein
      G¹, G², B^{a}, R and * are the same as defined above) with an oxidation solution which contains iodine, pyridine and water and a molar ratio of iodic acid to iodine (mole of iodic acid / mole of iodine) is 30 × 10⁻³ or less (hereinafter, referred to as "Oxidation solution of the present invention").
[2] The process according to [1] wherein the precursor having a phosphite triester bond represents a nucleic acid compound represented by formula (4): 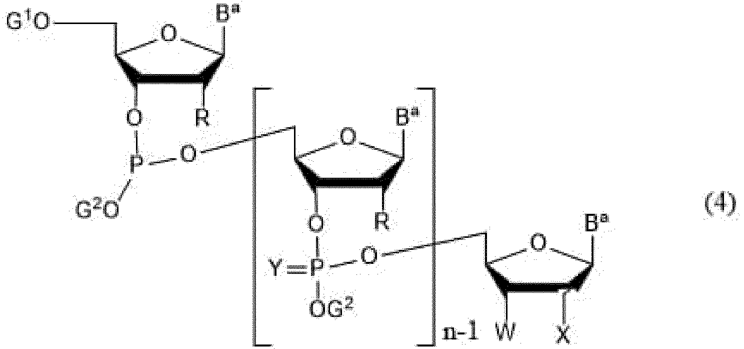 (wherein
   G¹ represents a protecting group for hydroxy group,
   G² is the same or different and each independently represents a protecting group for hydroxy group,
   B^{a} is the same or different and each independently represents a nucleic acid base which may be optionally protected with a protecting group,
   R is the same or different and each independently represents a protected hydroxy group, a hydrogen atom, a fluorine atom, a methoxy group, a 2-methoxyethyl group, or a OQ' group,
   Q' is the same or different and each independently represents a methylene group attached to a carbon atom at 4'-position of ribose, an ethylene group attached to a carbon atom at 4'-position of ribose, or an ethylidene group attached to a carbon atom at 4'-position of ribose,
   Y is the same or different and each independently represents an oxygen atom or a sulfur atom, n is any integer of 1 or more to 200 or less,
   when X represents OZ, W represents a OV group, and V represents a protecting group for hydroxy group,
   when X represents a R group, W represents a group represented by OZ,
   Z represents a group having a structure comprising a solid support and a connecting group, and
   when n is an integer of 2 or more, the nucleic acid compound represented by formula (4) may be incorporated by a non-nucleotide linker instead of at least one nucleotide between nucleotides at 5' terminus and 3' terminus of the nucleic acid compound),
   the compound containing a phosphate triester bond represents a compound represented by formula (5): 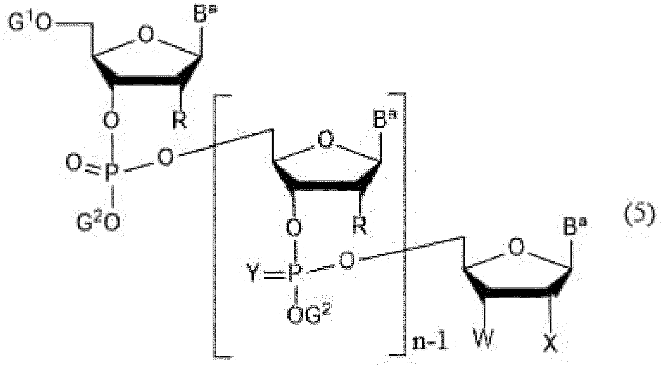 [wherein
      G¹, G², B^{a}, R, n, W, X, and Y are the same as defined above, and
      as defined in formula (4), a non-nucleotide linker may be incorporated instead of a nucleotide].
[3] The process for preparing nucleic acid oligomer according to [2], which comprises
   a step of elongating a chain strength of a nucleic acid compound represented by formula (5) to any chain length by an amidite method to obtain a nucleic acid represented by formula (5'): 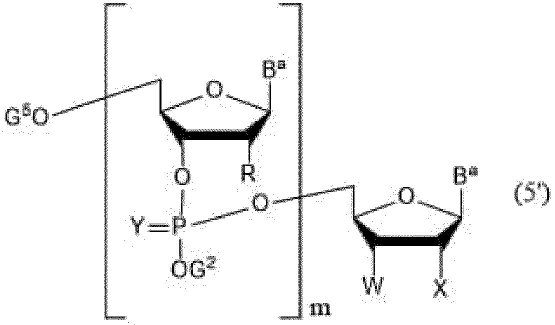 (wherein
   G², B^{a}, R, X and W are the same as defied in formula (5),
   G⁵ represents a protecting group for hydroxy group, or a hydrogen atom,
   m is an integer satisfying m≧n,
   Y is the same or different and each independently represents an oxygen atom or a sulfur atom, with the proviso that at least one of Y is an oxygen atom.),
   a step of cutting out the compound represented by formula (6): 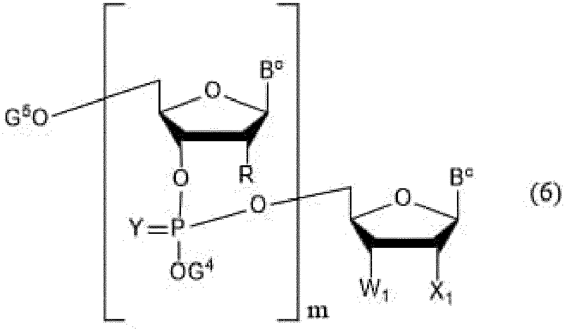 (wherein
      G⁵, R and m are the same as defined above,
      B^{c} is the same or different and each independently represents a nucleic acid base,
      G⁴ represents a hydrogen atom, an alkali metal ion, an ammonium ion, an alkyl ammonium ion, or a hydroxyalkyl ammonium ion,
      Y represents independently of each other an oxygen atom or a sulfur atom, and at least one thereof is an oxygen atom, and
      X₁ represents a hydroxy group, and W₁ represents a OV group wherein V represents a protecting group for hydroxy group, or
      X₁ represents a R group and W₁ represents a hydroxy group)
         from the compound represented by formula (5'),
      further a step of deprotecting the compound represented by formula (6) to prepare a deprotected nucleic acid oligomer represented by formula (7): 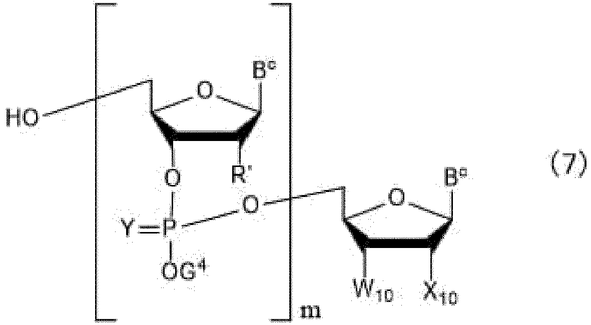 (wherein
         m, Y, G⁴, and B^{c} are the same as defined above,
         R' is the same or different and each independently represents a hydroxy group, a hydrogen atom, a fluorine atom, a methoxy group, a 2-methoxyethyl group, or a OQ' group,
         Q' is the same or different and each independently represents a methylene group attached to a carbon atom at 4'-position of ribose, an ethylene group attached to a carbon atom at 4'-position of ribose, or an ethylidene group attached to a carbon atom at 4'-position of ribose,
         X₁₀ and W₁₀ each represents independently of each other a hydroxy group, or
         X₁₀ represents an R' group, and W₁₀ represents a hydroxy group).
[4] The process according to the above [2] or [3] wherein the non-nucleotide linker is a linker composed by an amino acid backbone.
[5] The process according to [4] wherein the linker comprising an amino acid backbone is a linker having a structure selected from the following formulae (A14-1), (A14-2) and (A14-3). 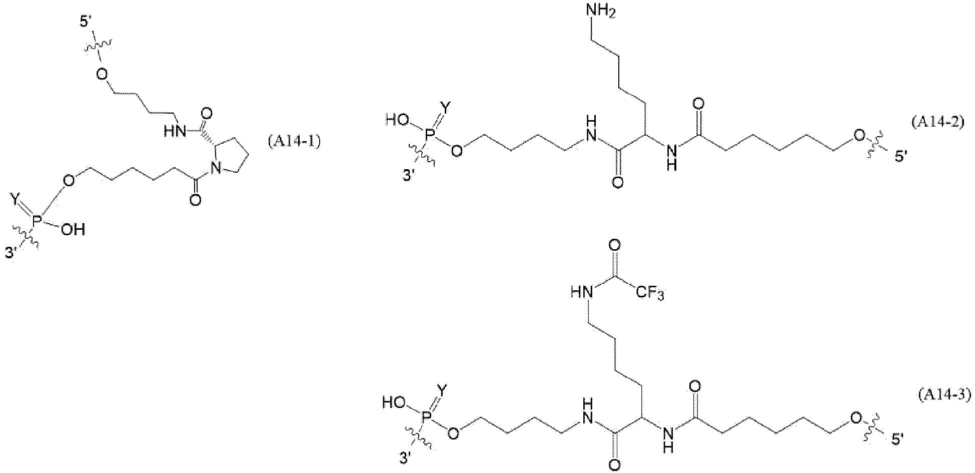
[6] The process according to any one of [1] to [5] wherein a concentration of iodine in an oxidation solution is 0.005 to 2 M.
[7] The process according to any one of [1] to [5] wherein a concentration of iodine in an oxidation solution is 0.005 to 0.2 M.
[8] The process according to any one of [1] to [5] wherein a concentration of iodine in an oxidation solution is 0.007 to 0.1 M.
[9] The process according to any one of [1] to [5] wherein a concentration of iodine in an oxidation solution is 0.008 to 0.07 M.
[10] The process according to any one of [1] to [9] wherein the oxidation solution is prepared by mixing iodine, pyridine and water.
[11] The process according to [10] wherein the oxidation solution is an oxidation solution further containing at least one solvent selected from a group consisting of acetonitrile and tetrahydrofuran.
[12] The process according to [10] or [11] wherein the oxidation solution is an oxidation solution further containing acetonitrile solvent.
[13] The process according to [11] wherein a solvent of the oxidation solution is a mixture of solvents which is obtained by mixing pyridine, water, acetonitrile, and tetrahydrofuran in a volume ratio of 1 to 90 : 1 to 50 : 0 to 90 : 0 to 90.
[14] The process according to [11] or [12] wherein a solvent of the oxidation solution is a mixture of solvents which is obtained by mixing pyridine, water and acetonitrile in a volume ratio of 1 to 90 : 1 to 50 : 0 to 90.
[15] The process according to any one of [1] to [14] wherein a molar ratio of iodic acid to iodine (mole of iodic acid / mole of iodine) is 25 × 10⁻³ or less.
[16] The process according to any one of [1] to [14] wherein a molar ratio of iodic acid to iodine (mole of iodic acid / mole of iodine) is 20 × 10⁻³ or less.
[17] The process according to any one of [1] to [14] wherein a molar ratio of iodic acid to iodine (mole of iodic acid / mole of iodine) is 15 × 10⁻³ or less.
[18] The process according to any one of [1] to [14] wherein a molar ratio of iodic acid to iodine (mole of iodic acid / mole of iodine) is 10 × 10⁻³ or less.
[19] The process according to any one of [1] to [14] wherein a molar ratio of iodic acid to iodine (mole of iodic acid / mole of iodine) is 5 × 10⁻³ or less.
[20] The process according to any one of [1] to [14] wherein a molar ratio of iodic acid to iodine (mole of iodic acid / mole of iodine) is 3 × 10⁻³ or less.
[21] The process according to any one of [1] to [20] wherein the oxidation solution is an oxidation solution having a duration from a preparation to a use in the oxidation reaction passed over one week or more.
[22] The process according to any one of [1] to [20] wherein the oxidation solution is an oxidation solution having a duration from a preparation to a use in the oxidation reaction passed over two weeks or more.
[23] The process according to any one of [1] to [22] wherein the nucleic acid is a ribonucleoside (RNA).
[24] The process according to any one of [2] to [23] wherein the nucleic acid is ribonucleoside (RNA), and its 2' protecting group is a protecting group represented by formula (12): 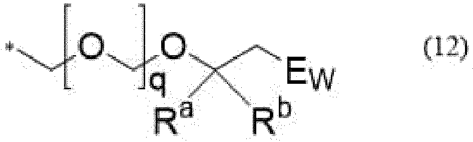 (wherein
   q is an integer of 1 to 5,
   R^{a} and R^{b} are the same or different and each independently represents a methyl group, an ethyl group, or a hydrogen atom,
   a bond marked with symbol * binds to an oxygen atom of OQ group, and
   E_{w} represents an electron-attracting group).
[25] The process according to [24] wherein R^{a} and R^{b} are a hydrogen atom at the same time, and E_{w} represents a cyano group.
[26] The process according to any one of [1] to [25] wherein the nucleic acid is a ribonucleoside (RNA) comprises 40 or more nucleotides in chain lengths.
[27] The process according to any one of [1] to [26], which further comprises a step of preparing an oxidation solution described in [1].

### Effect of Invention

The present invention provides an effective process for preparing a nucleic acid oligomer. According to a process of the present invention, an improvement in a purity of a nucleic acid oligomer prepared can be expected.

### Brief Description of Drawings

[Fig. 1]

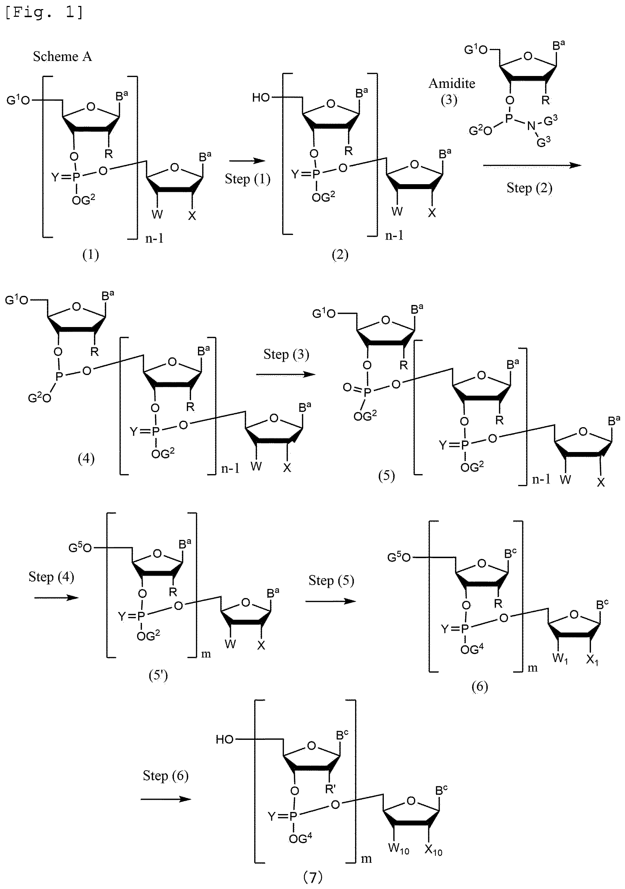

Figure 1 is a figure showing a scheme of the steps (1) to (6) in the process of the present invention.

### MODE FOR CARRYING OUT THE INVENTION

The following process is described: the process for preparing a nucleic acid compound having at its 5' terminus the nucleotide represented by the above formula (I) by a phosphoramidite method,
which comprises a step of reacting the precursor having a phosphite triester represented by the above formula (II) at a 5' terminus of the precursor with a oxidation solution containing iodine, pyridine and water and a molar ratio of iodic acid to iodine (mole of iodic acid / mole of iodine) is 30 × 10⁻³ or less (referred to as "oxidation solution of the present invention").

The following process is described: the process for preparing a nucleic acid compound comprising a step of reacting a nucleic acid precursor having a phosphite triester bond with an oxidation solution containing iodine, pyridine and water, and thereby the phosphite triester bond is converted into a phosphate triester bond, wherein a molar ratio of iodic acid to iodine (mole of iodic acid / mole of iodine) in the oxidation solution is 30 × 10⁻³ or less.

The molar ratio of iodic acid to iodine (mole of iodic acid / mole of iodine) in the oxidation solution of the present invention comprising iodine, pyridine and water is usually below 30 × 10⁻³ or less. The molar ratio of iodic acid to iodine (mole of iodic acid / mole of iodine) is preferably below 25 × 10⁻³ or less, further preferably below 20 × 10⁻³ or less, further more preferably below 15 × 10⁻³ or less, further more preferably below 10 × 10⁻³ or less, further more preferably below 5 × 10⁻³ or less, and further more preferably below 3 × 10⁻³ or less. Here the amounts of iodine and iodic acid are measured by ion chromatograph method described below.

The above-mentioned oxidation solution is typically prepared from iodine, water and pyridine. As the prepared solution, the solutions satisfying the above-mentioned mole ratio of iodic acid to iodine is used.

As a duration from a preparation of the oxidation solution to a synthesis of nucleic acid, the oxidation solution is stored at 25 to 60°C usually for 1 day or more, preferably for one week or more after the preparation, more preferably for two weeks or more after the preparation, further preferably for one month or more after the preparation, and any solutions having the above-prescribed mole ratio of iodic acid to iodine can be used. The storage temperature after preparation of the oxidation solution is not limited to the above-mentioned ranges, and the oxidation solution may be those which is stored at 0 to 80°C and also satisfies the above-prescribed requirements.

The concentration of iodine in the oxidation solution containing iodine, pyridine and water is adjusted to a range of usualy 0.005 to 2 M, preferably 0.005 to 0.2 M, more preferably 0.007 to 0.1 M, and further preferably 0.008 to 0.07 M.

The above-mentioned oxidation solution is typically prepared from iodine, water and pyridine, however at least one solvent selected from a group consisting of acetonitrile and tetrahydrofuran (THF) may be mixed in the oxidation solution, which may be used.

The solvents in the oxidation solution are any solvents that are produced by mixing 1 to 90 % by volume of pyridine, 1 to 50 % by volume of water, and 0 to 90 % by volume of acetonitrile, and 0 to 90 % by volume of tetrahydrofuran at an arbitrary ratio thereof to a total volume of the solution, and include preferably any solvents that are produced by mixing 5 to 90 % by volume of pyridine, 2 to 30 % by volume of water, 0 to 80 % by volume of acetonitrile, and 0 to 80 % by volume of tetrahydrofuran at an arbitrary ratio thereof. The stirring of the reaction system is not essential at preparing the oxidation solution, however usually the stirring is conducted within a range of 0.0 to 0.5 kW/m³ as the stirring power Pv, preferably 0.1 to 0.3 kW/m³.

The oxidation solution prepared according to the above-mentioned method can be stored by preparing those having a higher concentration of iodine than that when used in the oxidation reaction. The oxidation solution having high concentration thereof may be finally adjusted to a desired concentration of the iodine by diluting with the above-mentioned solvents, which may be used.

In the oxidation reaction of a phosphite triester bond, at least one kind of the compounds selected from a group consisting of N-methyl imidazole (NMI), N-methylmorpholine, lutidine, and triethylamine may be added to the above-mentioned oxidation solution. Alternatively, an iodide such as potassium iodine may be added thereto.

For the storage of the oxidation solution in a container, a glass container, a plastic container or a metallic container can be used. Examples of the plastic container include a container made of polyethylene and polypropylene and so on, and examples of the metallic container include a SUS container, or a hastelloy container.

The oxidation solution can be stored under air atmosphere or inert gas atmosphere, and examples of the inert gas that may be used include argon, nitrogen, carbon dioxide, and helium.

Examples of the compounds that contain a phosphorous (phosphite) triester bond include the compound of formula (4) above. Examples of the nucleic acid compound resulting from the reaction with the oxidation solution include the nucleic acid compound of formula (5).

In the formulae (4) and (5), examples of the compounds, wherein the Q' is the same or different and each independently represents a methylene group attached to a carbon atom at 4'-position of ribose, an ethylene group attached to a carbon atom at 4'-position of ribose, or an ethylidene group attached to a carbon atom at 4'-position of ribose, include the following structures of formula (8). 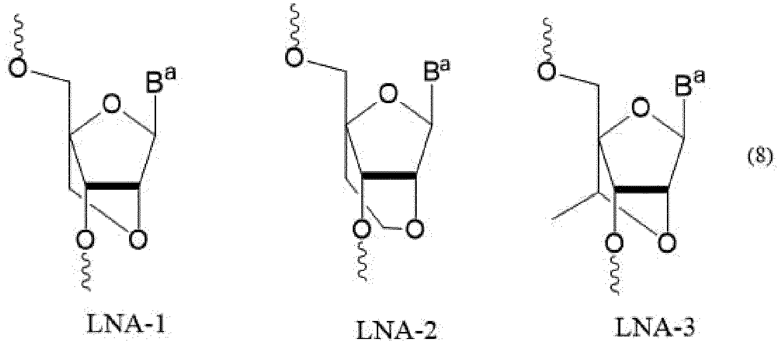 (wherein B^{a} represents a nucleic acid base which may be optionally protected.)

Examples of the group represented by Z include, a group comprising a solid support, and a connecting moiety that connects the solid support and an oxygen atom of a hydroxy group at 2'- or 3'-position of a ribose at 3' terminus of the nucleic acid oligomer, and more specifically, Z represents a structure represented by formula (9): 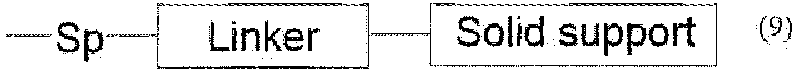 , in the formula (9), Sp represents a spacer.

Examples of the spacer (Sp) include the structure represented by the following formula (10).

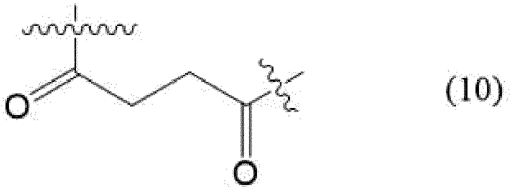

The Linker may for example, have a structure represented by the following formula (11), or a structure of the formula (11) wherein a hexamethlylene amine moiety is not contained in the structure represented by the following formula (11) and thus the aminopropyl group is linked to Si, or the Linker may have a structure represented by the following formula (15). 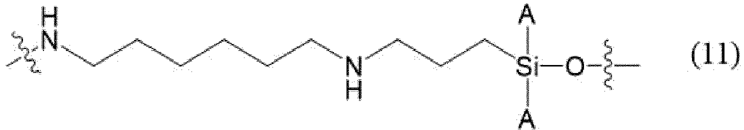 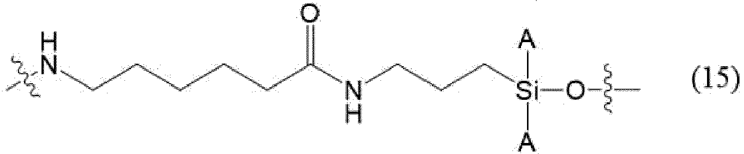 (wherein
A may be either a hydroxy group, an alkoxy group, or an alkyl group. Examples of the alkoxy group include a methoxy group, and an ethoxy group. Examples of the alkyl group include a methyl group, an ethyl group, an isopropyl group, a n-propyl group. Si represents that it binds to an oxygen atom of a hydroxy group on a support surface.)

Examples of the solid support include an inorganic porous support and organic resin support. Examples of the inorganic porous support include Controlled Pore Glass (CPG). Examples of the organic resin support include a support made of polystyrene.

Examples of the nucleoside (ribose and deoxyribose) contained in a nucleic acid oligomer to be used in the present invention include DNA, RNA, 2'-O-MOE (2-O-methoxymethyl), 2'-O-Me, 2'-F RNA, and the above LNA, and the above nucleoside is not limited thereto.

A synthesis method of nucleic acid oligomer comprising the oxidation step using the above oxidation solution by a solid phase synthesis comprises typically the following steps.
(1) a step of deprotecting the hydroxy group at 5'-position of a nucleoside having protected hydroxy which is bound to a solid support via a linker,
(2) a step of subjecting the resulting hydroxy group at 5'-position in the previous step to a coupling reaction with a phosphoramidite compound to obtain a phosphite triester compound,
(3) a step of preparing an elongated nucleic acid by oxidizing the phosphite triester produced in the previous step to a phosphate triester bond, or an optional step of converting the phosphite triester to a thiophosphate triester,
(4) a step of preparing a nucleic acid on the solid support, comprising repeating optional number of the reaction cycle of steps (1) to (3), that is, the deprotection step of a hydroxy group at 5'-position of the resulting nucleic acid, a coupling step of the hydroxy group at 5'-position with an amidite compound, and an oxidation step of the produced phosphite triester, and
(5) a step of cutting out the nucleic acid produced on the solid support in the above step (4), and then subjecting the resulting nucleic acid to a deprotection reaction, to liberate it from the solid support, and to prepare a deprotected nucleic acid oligomer,
wherein in the synthesis method of the nucleic acid oligomer above, the step (2) or (3), may be followed by a step of capping a hydroxy group at 5'-position which has not proceeded with a coupling reaction with a phosphoramidite compound, or the capping step may be added between any steps of the reaction cycle in the step (4).

More specifically, the above step (5) is carried out by subjecting a nucleic acid molecule on a solid support which is produced in step (4) to the following reaction steps (5-1) and (5-2) in the order thereof, and then subjecting the resulting reaction products to the reaction step (5-3). Here the reaction step (5-1) may be optionally conducted, and the reaction step (5-2) may be conducted according to a method described in JP patent No. 4705716. As a result, a nucleic acid oligomer wherein a protecting group is removed from a nucleic acid molecule liberated from the solid support, or a nucleic acid oligomer wherein a hydroxy group is protected can be produced.
(5-1) a reaction of deprotecting a protecting group for hydroxy group at 5' terminus of a nucleic acid molecule,
(5-2) a reaction of liberating the nucleic acid molecule by cutting out from a solid support, and
(5-3) a reaction of deprotecting a protecting group for a hydroxy group at 2'-position of a ribose of a nucleic acid molecule or at 3'-position of a ribose located at 3' terminus.

The schemes of the above steps (1) to (6) are shown in Figure 1. The oxidation reaction in the step (3) or the step (4) as depicted in Figure 1 is carried out by using the above oxidation solution. The definition of the substituents contained in chemical formula in Scheme A is the same as defined above.

The nucleic acid compound of formula (5) can be further elongated to any chain length by using a nucleotide type or a non-nucleotide type of linker according to an amidite method, and can be then used in a preparation of a nucleic acid compound represented by the above formula (5'). Also only a nucleic acid oligomer can be cut out from a nucleic acid compound which is bound to a solid support of the above formula (5') to obtain a nucleic acid oligomer represented by the above formula (6), and the resulting nucleic acid oligomer is then further deprotected to obtain a nucleic acid oligomer represented by the above formula (7) .

Hereinafter, substituent in each formula is described in more detail.

A nucleic acid base which may be optionally protected with a protecting group represented by B^{a} is not particularly limited. Examples of the nucleic acid base include adenine, cytosine, guanine, uracil, thymine, 5-methyl cytosine, pseudo uracil, 1-methyl pseudo uracil, and the others. Also the nucleic acid base may be optionally substituted with substituent(s). Examples of the substituent include a halogen atom (such as fluoro group, chloro group, bromo group, and iodo group), an acyl group (such as acetyl group), alkyl group (such as methyl group and ethyl group), arylalkyl group (such as benzyl group; alkoxy group (such as methoxyethyl group), cyanoalkyl group (such as cyanoethyl group), hydroxy group, acyloxymethyl group, amino group, monoalkylamino group, dialkylamino group, carboxy group, cyano group, and nitro group, as well as a combination of these two or more of the substituents.

In the case where a nucleic acid base contains an exocyclic amino group, the protecting group for the amino group is not particularly limited, and the publicly known protecting group used in a nucleic acid chemistry field may be used, and examples of the protecting group include benzoyl group, 4-methoxybenzoyl group, acetyl group, propionyl group, butyryl group, isobutyryl group, phenylacetyl group, phenoxyacetyl group, 4-tert-butylphenoxyacetyl group, 4-isopropylphenoxyacetyl group, and (dimethylamino)methylene group, as well as a combination of two or more of these protecting groups.

B^{a} represents more specifically any groups indicated below. 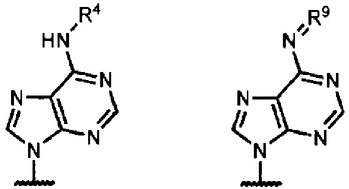 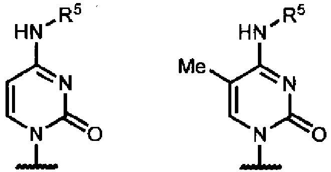 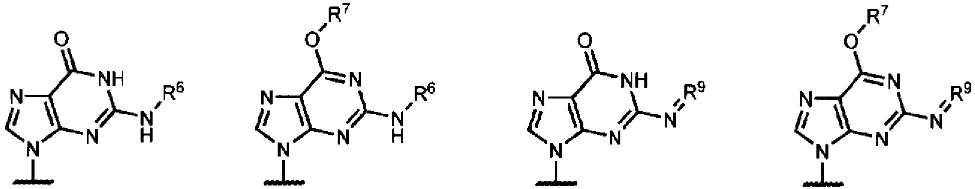 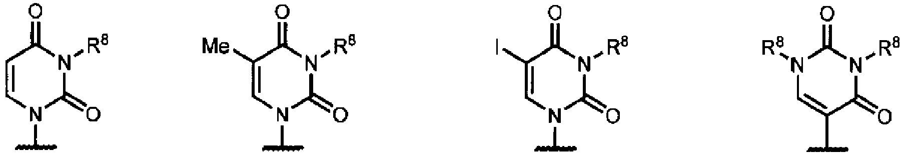 {wherein
R⁴ represents a hydrogen atom, a methyl group, a phenoxyacetyl group, a 4-tert-butylphenoxyacetyl group, a 4-isopropylphenoxyacetyl group, a phenylacetyl group, an acetyl group, or a benzoyl group,
R⁵ represents a hydrogen atom, an acetyl group, an isobutyryl group, or a benzoyl group,
R⁶ represents a hydrogen atom, a phenoxyacetyl group, a 4-tert-butylphenoxyacetyl group, a 4-isoproylphenoxyacetyl group, a phenylacetyl group, an acetyl group or an isobutyryl group,
R⁷ represents a 2-cyanoethyl group,
R⁸ represents a hydrogen atom, a methyl group, a benzoyl group, a 4-methoxybenzoyl group, or a 4-methylbenzoyl group, and
R⁹ represents a dimethylaminomethylene group.)

G¹ can be used without any particularly limitation as long as it can function as a protecting group, and a publicly known protecting group used for the amidite compound can be used widely

G¹ represents preferably the following groups. 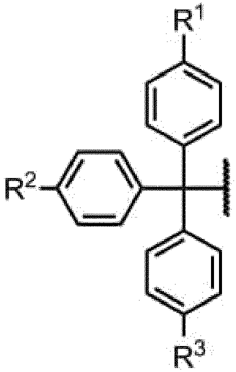 (wherein R¹, R² and R³ are the same or different and each independently represents a hydrogen atom or an alkoxy group.)

One of R¹, R² and R³ represents a hydrogen atom, and the remaining two thereof are the same or different (preferably the same) alkoxy group, and as an alkoxy group, a methoxy group is particularly preferred.

G² can be used without any particular limitation as long as it can function as a protecting group, and a publicly known protecting group for an amidite compound can be widely used. Examples of G² include an alkyl group, an alkenyl group, an alkynyl group, a cycloalkyl group, a haloalkyl group, an aryl group, a heteroaryl group, an arylalkyl group, a cycloalkenyl alkyl group, a cycloalkylalkyl group, a cyclylalkyl group, a hydroxyalkyl group, an aminoalkyl group, an alkoxyalkyl group, a heterocyclylalkenyl group, a heterocyclylalkyl group, a heteroarylalkyl group, a silyl group, a silyloxyalkyl group, a mono, di or tri- alkylsilyl group, a mono, di or tri-alkylsilyloxyalkyl group, and the others, and these groups may be optionally substituted with one or more electron-attracting group.

G² represents preferably an alkyl group substituted with electron-attracting group. Examples of the electron-attracting group include a cyano group, a nitro group, an alkylsulfonyl group, a halogen atom, an arylsulfonyl group, a trihalomethyl group, a trialkylamino group, and the others, and preferably a cyano group.

Particularly preferable example of G² include the following groups.

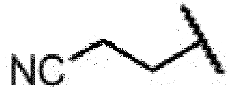

For G³, two G³ may be combined with each other to form a cyclic structure. Preferably, both G³ are an isopropyl group.

The alkyl group as the definitions of the above R¹, R², R³ and G² may be a straight chain or a branched chain, and preferably include an alkyl group containing 1 to 12 carbon atoms, and more preferably an alkyl group containing 1 to 6 carbon atoms. Specific examples of alkyl group include a methyl group, an ethyl group, a n-propyl group, an isopropyl group, a n-butyl group, an isobutyl group, a tert-butyl group, a n-pentyl group, an isopentyl group, and a hexyl group. An alkyl group part composed of the alkoxy group in the definition for above substituents has the same definition as that described in the definition of alkyl group described here.

Also, in the process of the present invention, an amidite compound can be used in a free state or a salt state thereof. Examples of salts of the amidite compound include a base addition salt or an acid addition salt, which are not particularly limited thereto. Examples of the base addition salt include salts with inorganic bases such as sodium, magnesium, potassium, calcium, aluminium and the others; salts with organic base such as methylamine, ethylamine, ethanolamine and the others; salts with basic amino acids such as lysine, ornithine, arginine, and the others); and ammonium salt. Specific examples of acid addition salts include mineral acids such as hydrochloric acid, hydrobromic acid, hydroiodic acid, sulfuric acid, nitric acid, phosphoric acid, and the others); acid addition salts, for example, salts with organic acids such as formic acid, acetic acid, propionic acid, oxalic acid, malonic acid, maleic acid, tartaric acid, fumaric acid, succinic acid, lactic acid, maleic acid, citric acid, methanesulfonic acid, trifluoromethansulfonic acid, ethanesulfonic acid, and the others, and salts with acidic amino acids such as aspartic acid, glutamic acid, and the others. Examples of the amidite compounds encompass salts, hydrates, solvates, crystal polymorphs, and the others.

When R represents a protected hydroxy group, its protecting group may be any one which can be used in an amidite method, and for example, the followings can be used: those described in WO 2013/027843 A1 and WO 2019/208571 A1, in addition to 2'-tert-butyl dimethylsilyl (TBDMS) group, 2'-bis(2-acetoxy)methyl (ACE) group, 2'-(triisopropylsilyloxy)methyl (TOM) group, 2'-(2-cyanoethoxy)ethyl (CEE) group, 2'-(2-cyanoethoxy)methyl (CEM) group, 2'-para-toluylsulfonylethoxymethyl (TEM) group, 2'-EMM group (WO 2006/022323 A1). Among 2' protecting group of these ribonucleoside (RNA), the above-mentioned protecting group represented by formula (12) is exemplified as a preferable protecting group. Further preferably, as the electron-attracting group represented by E_{w}, a protecting group containing a cyano group represented by formula (13) is exemplified. 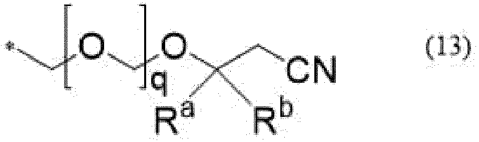 (wherein
q, R^{a} and R^{b} are the same as defined in the above formula (12), with the proviso that R^{a} and R^{b} do not represent a hydrogen atom at the same time.)

The protecting group represented by formula (13) can be synthesized, for example, according to a method described in WO 2013/027843 A1 and WO 2019/208571 A1, and the amidite compound having such a protecting group can be used in a preparation of a nucleic acid compound.

For an elongation reaction of nucleic acid, the amidite compound of formula (3) a shown in Scheme A of Figure 1 is used.

As non-nucleotide linker, a linker composed by amino acid backbone (for example, a linker composed by amino acid backbone as described in JP 5157168 B2 or JP 5554881 B2) is exemplified. Specifically, as a non-limiting example, a linker represented by formula (A14-1), formula (A14-2) or formula (A14-3) which are described for example in WO 2019/074110 is exemplified. In addition to these linkers, a particular linker as described in WO 2012/005368, WO 2018/182008 or WO 2019/074110 is exemplified.

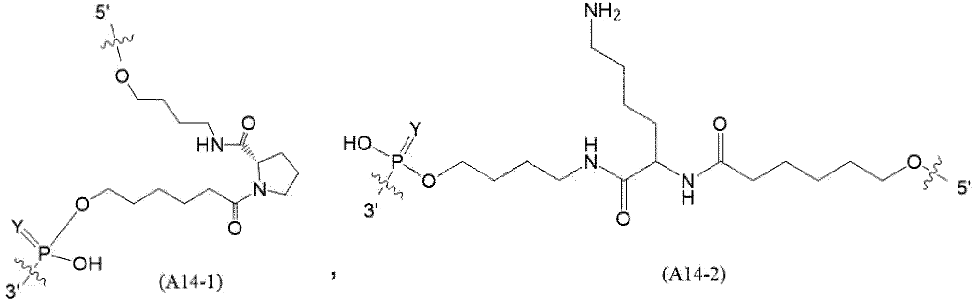

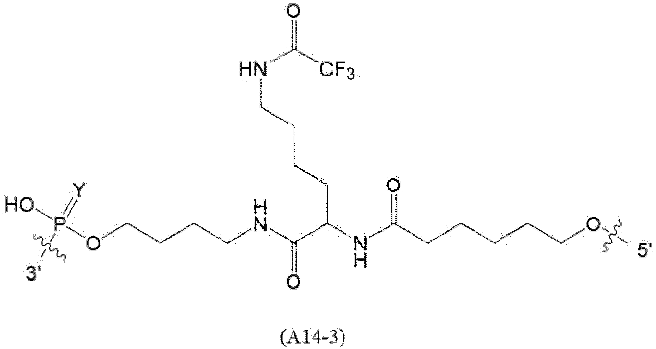

A nucleotide and an amidite wherein a R group in formula (3) and an R' group in formula (4) can be also prepared from nucleosides which are synthesized according to publicly known methods described in JP 3745226 B2 and so on, or WO 2001/053528, JP 2014-221817 A1 or publicly known methods referred to in these documents. Further, they can prepared by using a commercially available compound in line with the method described in the below Examples or methods with appropriate modifications to these methods.

For a synthesis of nucleic acid according to the amidite method in the above-mentioned steps (1) to (6), an elongation reaction of nucleic acid can be carried out by repeating each step such as a deprotection step and a condensation step, except for the oxidation step relating to the present invention in the step (3) in the scheme of Figure 1 according to a generally known method (for example, the method described in the above JP 5157168 B2 or JP 5554881 B2). Hereinafter, each step is described.

G⁴ represents a hydrogen atom, an alkali metal ion, ammonium ion, an alkyl ammonium ion, or a hydroxyalkyl ammonium ion. Examples of the alkali metal ion include a sodium ion, and a lithium ion. Also specific examples of alkyl groups include methyl, ethyl, n-propyl, isopropyl, n-butyl, isobutyl, tert-butyl, n-pentyl, isopentyl, and hexyl, and specific examples thereof include diethyl ammonium ion, triethyl ammonium ion, tetrabutyl ammonium ion, hexyl ammonium ion, dibutyl ammonium ion, and the others. Also as hydroxy alkyl ammonium ion, specific examples of hydroxyalkyl part thereof include hydroxymethyl, hydroxyethyl, hydroxy n-propyl, hydroxy isopropyl, hydroxy n-butyl, and tris hydroxy methyl, and more specific examples of hydroxyalkyl ammonium include tris hydroxymethyl ammonium ion and the others.

G⁵ represents a hydrogen atom or a protecting group, and when it represents a protecting group, it is the same as defined in G¹. When G⁵ is deprotected, it is a hydrogen atom, and the nucleotide compound in the case is also provided in a series of steps for nucleic acid elongation reaction.

### (Nucleic acid elongation reaction)

As used herein, "nucleic acid elongation reaction" means that a reaction for elongating oligonucleotide by attaching nucleotide sequentially through phosphodiester bond. The nucleic acid elongation reaction can be carried out according to the procedures of general phosphoramidite method. The nucleic acid elongation reaction may be carried out with a nucleic acid automatic synthesizer and so on which applies a phosphoramidite method.

The chain length of a nucleic acid oligomer may be, for example, 2 to 200 mer, and 10 to 150 mer, and 15 to 110 mer.

A 5' deprotection step in the step (1) is a step where a protecting group of a 5' hydroxyl group at RNA chain terminus which is supported on the solid support. As a general protecting group, 4,4'-dimethoxytrityl group (DMTr group), 4-monomethoxytrityl group, and 4,4',4"-trimethoxy trityl group are used. A deprotection reaction can be carried out by using an acid. Examples of the acid for deprotection reaction include trifluoroacetic acid, dichloroacetic acid, trifluoroethansulfonic acid, trichloroacetic acid, methansulfonic acid, trifluoromethansulfonic acid, trichloroacetic acid, methansulfonic acid, hydrochloric acid, acetic acid, p-toluenesulfonic acid, and the others.

The condensation step in the step (2) is a reaction where a nucleoside phosphoramidite represented by the following formula (3) as described in Scheme A of Figure 1 is attached to a 5' hydroxyl group at oligonucleotide chain terminus deprotected by the above deprotection step. As the phosphoramidite to be used in the nucleic acid elongation, an amidite compound represented by formula (3) or formula (A12) is used. Also, 2'-OMe, 2'-F, 2'-O-tert-butyldimetyl silyl group, 2'-O-methoxyethyl group, 2'-H, 2'-fluro-2'-deoxy-β-D-arabinofuranosyl and the others are included as another available phosphoramidite. As the above nucleoside phosphoramidite, those where 5' hydroxyl group is protected with a protecting group (for example, DMTr group) are used. The condensation step can be carried out by using an activator which activates the above-mentioned nucleotide phosphoramidite. Examples of the activator include 5-benzylthio-1H-tetrazole (BTT), 1H-tetrazole, 4,5-dicyanoimidazole (DCI), 5-ethylthio-1H-tetrazole (ETT), N-methyl benzimidazoliumtriflate (N-MeBIT), benzimidazoliumtriflate (BIT), N-phenylimidazoliumtriflate (N-PhIMT), imidazoliumtriflate (IMT), 5-nitrobenzimidazoliumtriflate (NBT), 1-hydroxybenzotriazole (HOBT), and 5-(bis-3,5-trifluoromethylphenyl)-1H-tetrazole, and the others.

The nucleotide phosphoramidite represented by formula (3) as described in Scheme A of Figure 1 (hereinafter, referred to as "amidite") is shown below.

A compound represented by formula: 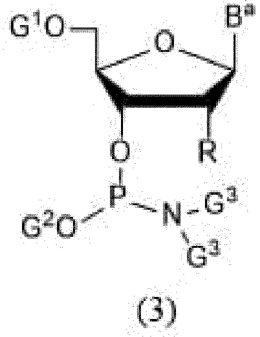 {wherein
G¹, G², G³, B^{a}, and R are the same as defined above.).

After the condensation step, as needed, the unreacted 5' hydroxyl group may be capped. The capping reaction can be carried out by using publicly known capping solution such as acetic anhydride-tetrahydrofuran solution, and phenoxy acetic anhydride / N-methyl imidazole solution.

The oxidation step of step (3) is a step for converting a phosphite group which is formed by the above condensation step into a phosphate group or a thiophosphate group. This step is a reaction of converting a trivalent phosphorus into pentavalent phosphorus using an oxidative agent, which can be carried out by acting an oxidation agent to oligonucleic acid derivatives supported on a solid support.

When a phosphite group is converted into a phosphate group, as "oxidation agent", for example, an iodine can be used. The oxidation agent can be used by adjusting a concentration thereof to 0.005 to 2 M. Water can be used as an oxygen source for oxidation, and pyridine, N-methyl imidazole (NMI), N-methyl morpholine, and triethylamine can be used as a base for proceeding with the reaction. Also the solvents are not particularly limited as long as they do not involve the reaction, and can be used as acetonitrile, tetrahydrofuran (THF) or a mixed solvents of these solvents at arbitrary ratio. For example, iodine/water/pyridine/acetonitrile, or iodine/water/pyridine, iodine/water/pyridine/NMI, or iodine/water/pyridine/THF can be used. The reaction temperature is preferably 5°C to 50°C. The reaction period of the reaction is usually appropriate to be 1 min. to 30 min. The amount of the reagent used is within a range of 1 to 100 mole(s), preferably 1 to 10 mole(s), as opposed to 1 mole of the compound supported on as solid support.

When a phosphite triester group is converted into a thiophosphate group, as "oxidation agent", for example, a sulfur, 3H-1,2-benzodithiol-3-one-1,1-dioxide (Beaucage reagent), 3-amino-1,2,4-dithiazole-5-thione (ADTT), 5-phenyl-3H-1,2,4-dithiazole-3-one (POS), [(N,N-dimethylaminomethyline)amino]-3H-1,2,4-dithiazoline-3-thione (DDTT), and phenylacetyldisulfide (PADS) can be also used. The oxidation agent can be used by diluting it with an appropriate solvent so as to adjust to 0.001 to 2 M. The solvents to be used are not particularly limited as long as they do not involve the reaction, and include, for example, dichloromethane, acetonitrile, pyridine, or any mixed solvents of these solvents. The oxidation step may be carried out after the above mentioned capping procedure, or vice versa, the capping procedure may be carried after the oxidation step, and the order of the procedures are not limited.

Among the step (5), in the step of deprotecting a protecting group for a phosphorus group, when a synthesis of a nucleic acid having a desirable sequence is completed, an amine compound is acted to deprotect a protecting group of a phosphorus part. Examples of the amine compound include, for example, diethylamine as described in JP 4705716 B2 and the others.

The protecting group for 5' hydroxyl group of a nucleoside introduced in the last stage of an elongation may be used for a column purification with 5' protecting group as a tag after the below-mentioned procedures of a cutting out from a solid support and a deprotection of a protecting group, or the protecting group for 5' hydroxyl group may be deprotected after the column purification.

In the step (5), the cutting out of a nucleic acid oligomer which is elongated to a desirable chain on a solid support from a solid support is usually carried out by using a concentrated ammonium water as a cutting out agent.

Further, using ammonia or amine compound or the others, for example, an oligo nucleotide chain is recovered by cutting it out from a solid support. Examples of the amine compound include methylamine, ethylamine, isopropylamine, ethylenediamine, diethylamine, and the others.

In the step (6), a protecting group for hydroxy group at 2 position or 3 position of ribose of the nucleic acid compound (6) which is cut out from a solid support in the step (5) can be removed according to a method described in WO 2006/022323 A1, WO 2013/027843 A1, or WO 2019/208571 A1 to obtain a deprotected nucleic acid oligomer (7).

Examples of the nucleic acid oligomer which can be prepared according to the process of the present invention include those wherein a nucleoside contained in the nucleic acid oligomer is RNA, DNA, as well as RNA having 2'-O-MOE, 2'-O-Me, 2'-F, and LNA, which is not limited thereto. For example, various nucleosides described in Xiulong, Shen et al., Nucleic Acids Research, 2018, Vol. 46, No.46, 1584-1600, and Daniel O'Reilly et al., Nucleic Acids Research, 2019, Vol. 47, No.2, 546-558 are included.

As typical examples of nucleic acid oligomer which can be used in the process of the present invention, the following examples are indicated in addition to examples described in working examples, which are not limited thereto.

Hereinafter, in a description of a sequence, u is uridine, c is cytidine, A is adenosine, and G is guanosine.

A nucleic acid oligomer having the following sequences (B) and (C) as described in WO 2019/060442 is exemplified.
Sequence (B): 5'-AUGGAAUmACUCUUGGUUmACdTdT-3' (Antisense) (Sequence No. 3) 21 mer
Sequence (C): 5'-GUmAACmCmAAGAGUmAUmUmCmCmAUmdTdT-3' (Sense) (Sequence No. 4) 21 mer

In the sequence (B) and sequence (C), Um is 2'-O-metyluridine, Cm is 2'-O-methylcytidine, and dt is thymidine.

A nucleic acid oligomer as described in Daniel O'Reilly et al., Nucleic Acids Research, 2019, Vol. 47, No.2, 546-558 (refer to p. 553) is exemplified. Typical examples thereof include a nucleic acid oligomer having the following sequence (D).
Sequence (D): 5'-AGAGCCAGCCUUCUUAUUGUUUUAGAGCUAUGCUGU-3' (Sequence No. 5) 36 mer

A nucleic acid oligomer as described in JP 4965745 B2 is exemplified. Typical examples thereof include a nucleic acid oligomer having the following sequence (E).
Sequence (E): 5'-CCAUGAGAAGUAUGACAACAGCC-P-GGCUGUUGUCAUACUUCUCAUGGUU-3' 49 mer. CCAUGAGAAGUAUGACAACAGCC (Sequence No. 6), GGCUGUUGUCAUACUUCUCAUGGUU (Sequence No. 7).

In the Sequence (E), "P" is depicted by a partial structure separated by wavy lines in the following formula (A5) .

A nucleic acid oligomer having the following sequence (F) as described in Nucleic Acids Research, 2019, Vol. 47, No. 2: 547 is exemplified.
Sequence (F): 5'-ACAGCAUAGCAAGUUAAAAUAAGGCUAGUCCGUUAUCAACUUGAAAAAGUGGCACCGAG UCGGUGCU-3' (Sequence No. 8) 67 mer

A nucleic acid oligomer having the following sequence (G) as described in JP 2015-523856, 173 is exemplified.
Sequence (E): 5'-GUUUUCCCUUUUCAAAGAAAUCUCCUGGGCACCUAUCUUCUUAGGUGCCCUCCCUUGUU UAAACCUGACCAGUUAACCGGCUGGUUAGGUUUUU-3' (Sequence No. 9) 94 mer

A nucleic acid oligomer as described in JP 2017-537626 is exemplified. Typical examples thereof include a nucleic acid oligomer having the following sequences (F), (G), (H), and (J).
Sequence (F): 5'-AGUCCUCAUCUCCCUCAAGCGUUUUAGAGCUAGUAAUAGCAAGUUAAAAUAAGGCUAGU CCGUUAUCAACUUGAAAAAGUGGCACCGAGUCGGUGCUUUU-3' (Sequence No. 10) 100 mer
Sequence (G): 5'-GCAGAUGUAGUGUUUCCACAGUUUAAGAGCUAUGCUGGAAACAGCAUAGCAAGUUUAAA UAAGGCUAGUCCGUUAUCAACUUGAAAAAGUGGCACCGAGUCGGUGCUUUUUUU-3' (Sequence No. 11) 113 mer
Sequence (H): 5'-dAdGdTdCdCdTdCdAdTdCdTdCdCdCdTdCdAdAdGdCGUUUAAGAGCUAUGCUGGU AACAGCAUAGCAAGUUUAAAUAAGGCUAGUCCGUUAUCAACUUGAAAAAGUGGCACCGA GUCGGUGCUUUUUUU-3' (Sequence No. 12) 113 mer

In the sequence (H), dT is thymidine, dC is 2'-deoxycytidine, dA is 2'-deoxyadenosine, and dG is 2'-deoxyquanosine.
Sequence (J): 5'-AmsGmsUmsCCUCAUCUCCCUCAAGCGUUUAAGAGCUAUGCUGGUAACAGCAUAGCAAG UUUAAAUAAGGCUAGUCCGUUAUCAACUUGAAAAAGUGGCACCGAGUCGGUGCUUUUms UmsUmsU-3' (Sequence 13) 113 mer

In the Sequence (J), Um is 2'-O-methyluridine, Am is 2'-O-methyladenosine, Gm is 2'-O-methylquanosine, or s is phosphorothioate modification.

### EXAMPLES

Hereinafter, the resent invention is explained in more detail by working examples, and the present invention is not limited to these examples.

### <Measurement method>

Firstly, various measurement methods used in the following tests are shown below.

A purity of oligonucleotide was measured by HPLC.

A HPLC measurement condition is shown in the below Table 1.

### (Measurement method 1: Measurement of oligonucleotide purity)

**[Table 1]**

| Column | ACQUITY UPLC Oligonucleotide BEH |
|---|---|
| | C18, 2.1 mm × 100 mm, 1.7 µm × two columns |
| Flow rate | 0.2 mL/min |
| Detection wavelength | 260 nm |
| Mobile phase A | 100 mM aqueous hexylamine acetate (pH = 7.0) |
| Mobile phase B | 500 mM aqueous hexylamine acetate : acetonitrile = 1:4(v) |
| Gradient condition | Bconc. 43% (0 min)-56%(30 min) -90% (30.01 min)-90%(35 min) -43%(35.01 min)-43%(50 min) |
| Column temperature | 80°C |

### (Measurement method 2: Measurement of oligonucleotide yield)

OD₂₆₀ of the above crude product was measured. OD₂₆₀ represents an absorbance at UV260 nm per 10 mm optical path length in a 1 mL solution (pH = 7.5). Since it is generally known that 1 OD = 40 µg for RNA, the yield was calculated based on the above measured value of OD₂₆₀.

### (Measurement method 3: Measurement of iodic acid concentration)

A measurement of iodic acid was conducted by ion chromatograph method. The concentration thereof was calculated by comparing with a standard sample (NaIO₃). A measurement condition of the ion chromatograph method is shown in the below Table 2.

**[Table 2]**

| Column | IONPac AS18 4 mm × 250 mm |
|---|---|
| Flow rate | 0.8 mL/min |
| Detector | Electrical conductivity detector |
| Eluate | KOH |
| Gradient condition | 5 mM (0 min)-5 mM (8 min)-60 mM(8.1 min)-60 mM (25 min) |
| Suppressor | ASRS500 4mm 149 mA |
| Column temperature | 30°C |

### (Measurement method 4: Measurement of iodine concentration)

A measurement of iodine was conducted by ion chromatograph method. The concentration thereof was calculated by comparing with a standard sample (I₂). A measurement condition of the ion chromatograph method is shown in the below Table 3.

**[Table 3]**

| Column | IONPac AS11-HC 4 mm × 250 mm |
|---|---|
| Flow rate | 0.8 mL/min |
| Detector | Electrical conductivity detector |
| Eluate | KOH |
| Gradient condition | 60 mM isocratic |
| Suppressor | ASRS500 4mm 119 mA |
| Column temperature | 30°C |

### <Solid phase synthesis of oligonucleotide)

Sequence (I): 5'-AGCAGAGUACACACAGCAUAUACC-P-GGUAUAUGCUGUGUGUACUCUGCUUC-P-G-3' (Sequence Nos. 1, 2) 53 mer

In the above sequence (I), "A" is represented by a partial structure separated by wavy lines in the following formula (A1). "C" is represented by a partial structure separated by wavy lines in the following formula (A2). "G" is represented by a partial structure separated by wavy lines in the following formula (A3). "U" is represented by a partial structure separated by wavy lines in the following formula (A4). "P" is represented by a partial structure separated by wavy lines in the following formula (A5). Here "A" at a 5' terminus is represented by a partial structure separated by wavy lines in the following formula (A6). Also "G" at a 3' terminus is represented by a partial structure separated by wavy lines in the following formula (A7).
AGCAGAGUAC ACACAGCAUA UACC (Sequence No. 1)
GGUAUAUGCU GUGUGUACUC UGCUUC (Sequence No. 2)

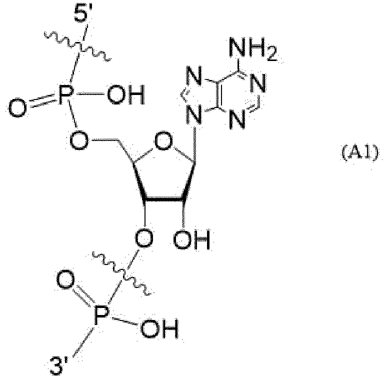

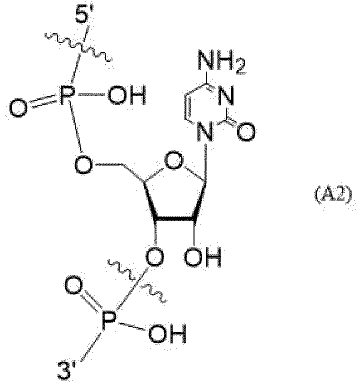

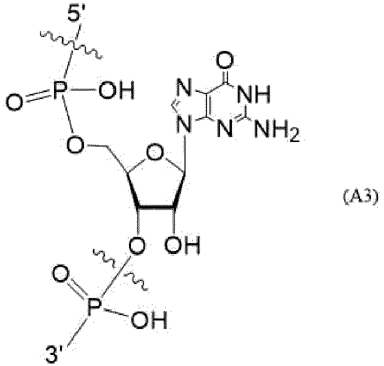

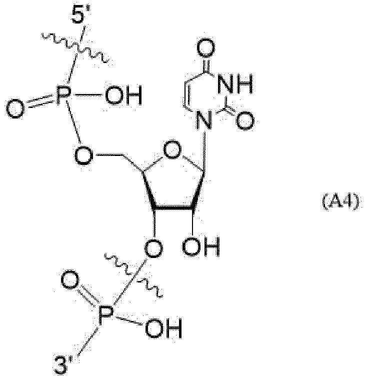

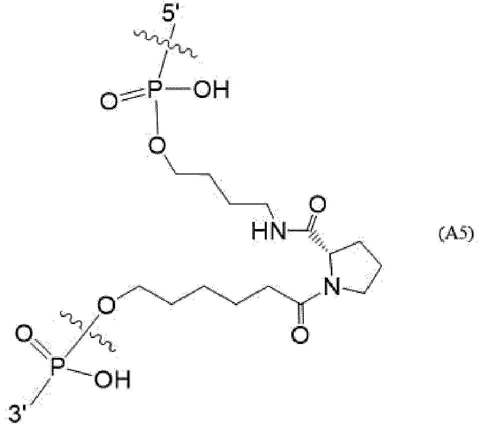

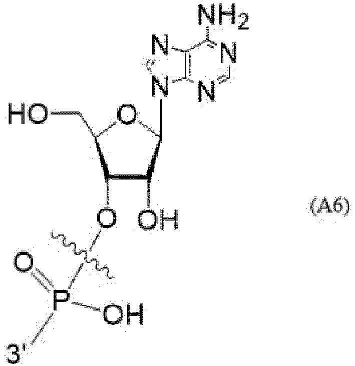

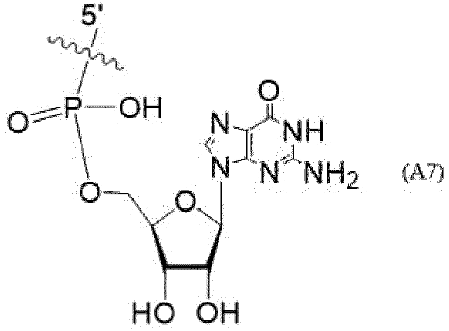

Using a Controlled Pore Glass (CPG) as a solid support and a NTS M-4MX-E (manufactured by NIHON TECHNO SERVICE CO. LTD) as a nucleic acid synthesizer, according to a phosphoramidite solid phase synthesis method, an oligonucleotide composed by the above sequence (I) was synthesized from the 3' side to the 5' side. The synthesis was carried out on a scale of about 1 µmol scale when the NTS M-4MX-E (manufactured by NIHON TECHNO SERVICE CO. LTD) was used, and was carried out on a scale of about 80 µmol scale when the AKTA oligopilot plus100 (manufactured by GE healthcare). Also in the synthesis, a uridine EMM amidite as described in Example 2 of US 2012/0035246, a cytidine EMM amidite as described in Example 3 thereof, an adenosine EMM amidite as described in Example 4 thereof, a guanosine EMM amidite as described in Example 5 thereof, and the compound (3) as described in WO 2017/188042 were used, and a high-purity solution of trichloro acetic acid in toluene was used as a deblocking solution, and 5-benzylmercapto-1H-tetrazole as a condensation agent, and iodine solution was used as an oxidation agent, and a phenoxy acetic anhydride solution and a N-methyl imidazole solution was used as a capping solution,

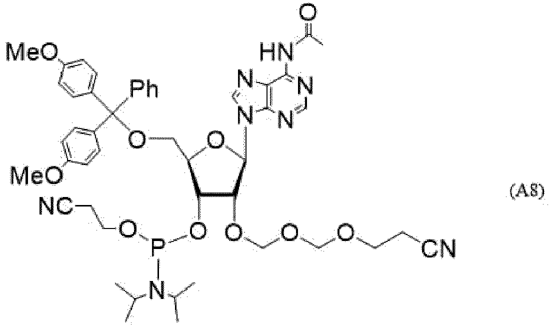

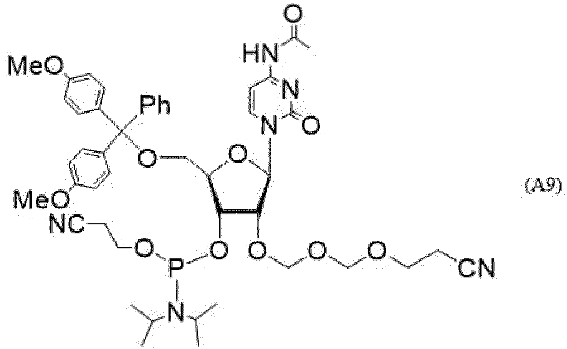

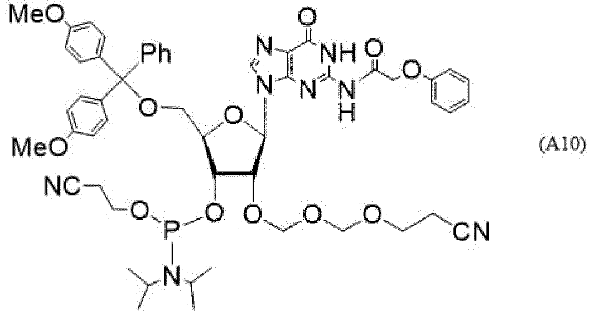

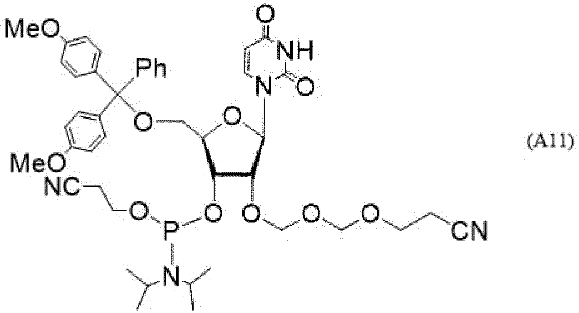

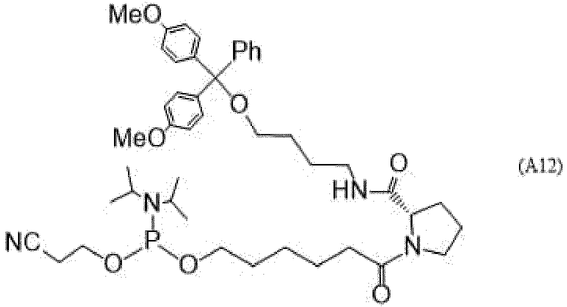

Next, specific preparation examples of nucleic acid oligomer prepared by the process of the present invention are shown. Here, in the following examples, the oligonucleotides prepared according to the process of the present invention are nucleic acid oligomer having the sequence (I) showin in the above sequence Nos. 1 and 2.

Also, the guanosine derivatives as described in the following Examples and Comparative Examples represent the compounds represented by the following structural formula. A circle as depicted in the following structural formula represents CPG schematically.

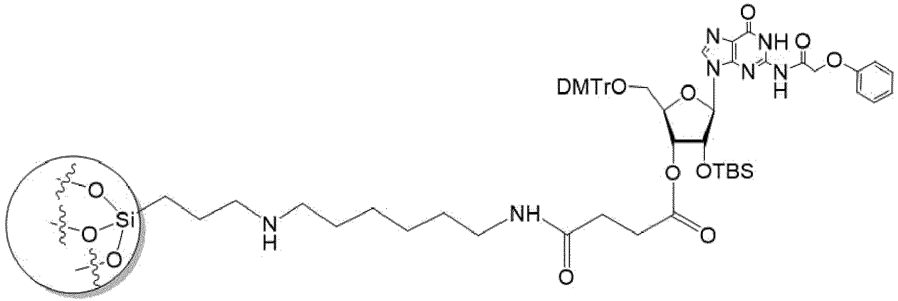

### (Example 1)

Using Controlled Pore Glass (CPG) on which 1.08 µmol guanosine derivative was supported, and each amidite represented by formula (A8), formula (A9), formula (A10) formula (A11), or formula (A12) respectively, the nucleic acid oligomer represented by sequence (I) was automatically synthesized by NTS M-4MX-E (manufactured by NIHON TECHNO SERVICE CO. LTD) from the 3'-side to the 5'-side. For procedures for automatic synthesis, firstly 1.4 mL of 3% trichloroacetic acid solution in toluene at each time was liquid-transferred to CPG, a trityl protecting group at 5' position was deprotected, and subsequently, 0.3 mL of each amidite, and 0.4 mL of 5-benzylmercapto-1H-tetrazole as a condensation agent were liquid-transferred to CPG, and a coupling reaction was proceeded on a hydroxyl group at 5'-position. Subsequently, after a solution of acetonitrile: water : pyridine = 58.2 : 34.4 : 7.2 (% by weight) containing 11 mM iodine was prepared, 0.7 mL of the solution stored at 25°C for 2 years was liquid-transferred, thereby a phosphite group was converted into a phosphate group. The molar concentration of iodine in the oxidation solution when used in a nucleic acid synthesis can be measured according to the measurement method 4, and the concentration thereof was 11 mM, and the concentration of iodic acid can be measured according to the measurement method 3, and the concentration thereof was 0.029 mM. That is, the ratio of molar concentration of iodic acid relative to the molar concentration of iodine in the oxidation solution when used in the synthesis was 2.6 × 10⁻³. Successively, a 0.5 mL of 0.1 M phenoxy acetic anhydride solution in acetonitrile and a 0.5 mL of 10% N-methyl imidazole / 10% 2,6-lutidine solution in acetonitrile were used, thereby a capping reaction was carried out at the reaction point where the coupling reaction did not proceed. Further, these steps were repeated 52 times in total, thereby a nucleic acid oligonucleotide of a sequence represented by sequence (I) was synthesized on a CPG support, and a trityl protecting group at 5'-position was deprotected with a 3% trichloroacetic acid solution in toluene. After that, a CPG support on which total amounts of oligonucleotide was supported was treated with 752 µL ammonia water and 252 µL ethanol, and a nucleic acid oligomer was liberated from the solid support, and ammonia water and ethanol were removed by nitrogen-spraying. Next, the liberated oligonucleotide was solubilized in 400 µL dimethylsulfoxide, and nitromethane 5.3 µL and a stir bar were added thereto, and then a 530 µL (10.2 mole of TBAF per 1 mole of protecting group) of solution of 1M tetra-n-butyl ammonium fluoride (TBAF) in dimethyl sulfoxide, which was subjected to a dehydration treatment with molecular sieve 4A was inflowed thereto at 30°C under stirring with a stirrer, and the resulting mixture was kept warm for 4 hours to deprotect a protecting group of 2'-EMM protecting group. The nucleic acid oligomer was obtained by a precipitation operation. As a result of measurement according to the measurement method 2, yields were 9.1 mg, and as a result of measurement according to the measurement method 1, the purity was 61 %.

### (Example 2)

A nucleic acid oligomer of sequence (I) was obtained in a similar manner to that of the experiment in Example 1 except that the followings were changed: a scale was set to the particular scale when Controlled Pore Glass (CPG) on which 78.20 µmol guanosine derivative was supported was used, and AKTA oligonucleotide plus 100 (manufactured by GE healthcare) was used, and the solution described in Table 2 was used as an oxidation solution containing iodine, and after a completion of nucleic acid synthesis procedure, a CPG support on which 20.13 µmol of oligonucleotide was supported was collected, and the nucleic acid oligomer was liberated from the solid phase carrier by using 7.5 mL of ammonia water and 2.5 mL of ethanol, and was concentrated by an evaporator to remove ammonia water and ethanol, and next, the liberated oligonucleotide was solubilized in 8.0 mL of dimethyl sulfoxide, and nitromethane 106 µL and a stirrer bar were added thereto, and 10.6 mL (the amount of TBAF was 10.2 mol per 1 mole of the protecting group) of 1M solution of tetra-n-butyl ammonium fluoride (TBAF) in dimethyl sulfoxide which was subjected to a dehydration treatment with molecular sieve 4A was used. Yields were 170.5 mg, and the purity was 60 %.

### (Example 3)

A nucleic acid oligomer of sequence (I) was obtained in a similar manner to that of the experiment in Example 1 except that Controlled Pore Glass (CPG) on which 1.10 µmol guanosine derivative was supported was used, and the solution described in Table 2 was used as an oxidation solution containing iodine. Yields were 8.4 mg, and the purity was 60 %.

### (Example 4)

A nucleic acid oligomer of sequence (I) was obtained in a similar manner to that of the experiment in Example 2 except that the followings were changed: the solutions described in the Table below were used as an oxidation solution, and a scale was set to the particular scale when Controlled Pore Glass (CPG) on which 78.20 µmol guanosine derivative was supported was used, and the oxidation solution described in Table 2 was used as an oxidation solution containing iodine, and after a completion of nucleic acid synthesis procedure, a CPG carrier on which 20.16 µmol of oligonucleotide was supported was collected. Yields were 180.0 mg, and the purity was 59 %.

### (Example 5)

A nucleic acid oligomer of sequence (I) was obtained in a similar manner to that of the experiment in Example 1 except that Controlled Pore Glass (CPG) on which 1.06 µmol guanosine derivative was supported was used, and the solution described in Table 2 was used as an oxidation solution containing iodine. Yields were 9.1 mg, and the purity was 56 %.

### (Example 6)

A nucleic acid oligomer of sequence (I) was obtained in a similar manner to that of the experiment in Example 2 except that the solution described in Table 2 was used as an oxidation solution containing iodine, and after a completion of nucleic acid synthesis procedure, a CPG carrier on which 20.20 µmol of oligonucleotide was supported was collected. Yields were 154.8 mg, and the purity was 57 %.

### (Example 7)

A nucleic acid oligomer of sequence (I) was obtained in a similar manner to that of the experiment in Example 1 except that Controlled Pore Glass (CPG) on which 1.09 µmol guanosine derivative was supported was used and the solution described in Table 2 was used as an oxidation solution containing iodine. Yields were 9.2 mg, and the purity was 49 %.

### (Example 8)

A nucleic acid oligomer of sequence (I) was obtained in a similar manner to that of the experiment in Example 1 except that Controlled Pore Glass (CPG) on which 1.04 µmol guanosine derivative was supported was used and the solution described in Table 2 was used as an oxidation solution containing iodine. Yields were 7.7 mg, and the purity was 46 %.

### (Example 9)

A nucleic acid oligomer of sequence (I) was obtained in a similar manner to that of the experiment in Example 1 except that Controlled Pore Glass (CPG) on which 1.00 µmol guanosine derivative was supported was used and the solution described in Table 2 was used as an oxidation solution containing iodine. Yields were 7.9 mg, and the purity was 42 %.

### Reference Example 1

A nucleic acid oligomer of sequence (I) was obtained in a similar manner to that of the experiment in Example 1 except that Controlled Pore Glass (CPG) on which 1.04 µmol guanosine derivative was supported was used and the solution described in Table 2 was used as an oxidation solution containing iodine. Yields were 7.8 mg, and the purity was 35 %.

The results of Examples 1 to 9 and Reference Example 1 are shown in Table 4.

**[Table 4]**

| | Oxidation solution | | | | | | | | Nucleic acid synthesis |
|---|---|---|---|---|---|---|---|---|---|
| | I₂ (mM) | Pyrid ine (wt%) | Water (wt%) | Aceto nitri le (wt%) | HIO₃ (mM) | I₂/HIO₃ molar ratio | Storag e durati on | Stora ge Temp. | Nucleic acid purity (%) |
| Ex.1 | 11.04 | 7.2 | 34.3 | 58.2 | 0.029 | 2.6×10⁻³ | 2 years | 25°C | 61 |
| Ex.2 | 11.04 | 7.2 | 34.3 | 58.2 | 0.029 | 2.6×10⁻³ | 2 years | 25°C | 60 |
| Ex. 3 | 11.22 | 7.2 | 34.3 | 58.2 | 0.057 | 5.1×10⁻³ | 1 year | 25°C | 60 |
| Ex. 4 | 11.78 | 7.2 | 34.3 | 58.2 | 0.091 | 7.7×10⁻³ | 8 months | 25°C | 59 |
| Ex. 5 | 11.24 | 7.2 | 34.3 | 58.2 | 0.113 | 10.1×10⁻³ | 6 weeks | 25°C | 56 |
| Ex. 6 | 50.73 | 88.7 | 10.0 | 0.0 | 0.709 | 14.0×10⁻³ | 8 months | 25°C | 57 |
| Ex. 7 | 10.11 | 7.2 | 34.3 | 58.2 | 0.193 | 19.1×10⁻³ | 2 weeks | 25°C | 49 |
| Ex. 8 | 10.43 | 7.2 | 34.3 | 58.2 | 0.238 | 22.8×10⁻³ | 1 week | 25°C | 46 |
| Ex.9 | 50.08 | 88.7 | 10.0 | 0.0 | 1.441 | 28.8×10⁻³ | 24 hours | 25°C | 42 |
| Ref. Ex.1 | 9.89 | 7.2 | 34.3 | 58.2 | 0.353 | 35.7×10⁻³ | 24 hours | 25°C | 35 |

As shown in the above table, when the oxidation solution of the present invention having certain level of 30 × 10⁻³ or less of a molar ratio of iodic acid to iodine was used, a nucleic acid oligomer having high purity was obtained compared to the case of using the oxidation solution of the Reference example 1.

### [Industrial Applicability]

The present invention provides an efficient process for preparing nucleic acid oligomer. Also, an improvement in a purity of a nucleic acid oligomer that is prepared according to a process for preparing nucleic acid oligomer can be expected.

### [Sequence Listing Free Text]

Sequence Nos. 1 to 13 in a sequence listing represent a nucleotide sequence of oligonucleotide that is prepared according a process of the present invention.

## Claims

1. A process for preparing a nucleic acid compound having at its 5'-terminus a nucleotide represented by formula (I): 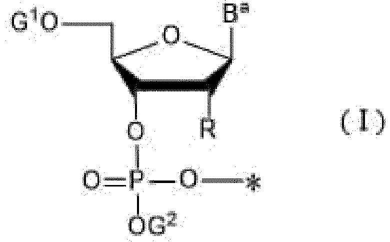 (wherein
G¹ and G² each represents independently of each other a protecting group for hydroxy group, and B^{a} represents a nucleic acid base which may be optionally protected with a protecting group,
R represents a protected hydroxy group, a hydrogen atom, a fluorine atom, a methoxy group, a 2-methoxyethyl group, or a OQ' group,
Q' represents a methylene group attached to a carbon atom at 4'-position of ribose, an ethylene group attached to a carbon atom at 4'-position of ribose, or an ethylidene group attached to a carbon atom at 4'-position of ribose, and
a bond marked with symbol * represents a bond directing to 3' terminus side of a nucleic acid) by a phosphoramidite method,
which comprises a step of reacting a precursor having a phosphite triester bond represented by a formula (II): 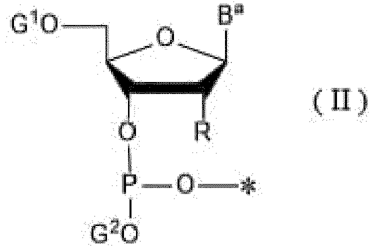 (wherein
G¹, G², B^{a}, R and * are the same as defined above) with an oxidation solution which contains iodine, pyridine and water and a molar ratio of iodic acid to iodine (mole of iodic acid / mole of iodine) is 30 × 10⁻³ or less.

2. The process according to claim 1 wherein the precursor having a phosphite triester bond represents a nucleic acid compound represented by formula (4): 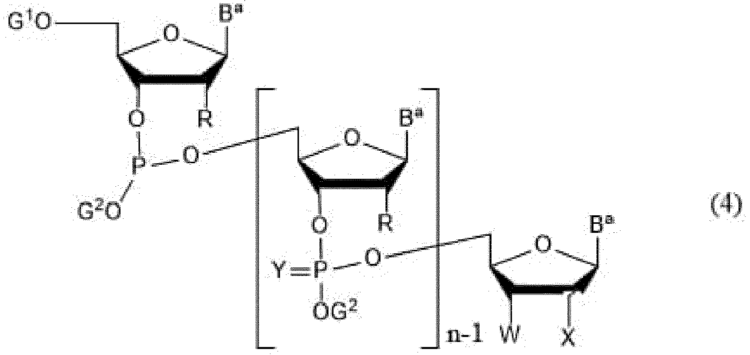 (wherein
G¹ represents a protecting group for hydroxy group,
G² is the same or different and each independently represents a protecting group for hydroxy group,
B^{a} is the same or different and each independently represents a nucleic acid base which may be optionally protected with a protecting group,
R is the same or different and each independently represents a protected hydroxy group, a hydrogen atom, a fluorine atom, a methoxy group, a 2-methoxyethyl group, or a OQ' group,
Q' is the same or different and each independently represents a methylene group attached to a carbon atom at 4'-position of ribose, an ethylene group attached to a carbon atom at 4'-position of ribose, or an ethylidene group attached to a carbon atom at 4'-position of ribose,
Y is the same or different and each independently represents an oxygen atom or a sulfur atom,
n is any integer of 1 or more to 200 or less,
when X represents OZ, W represents a OV group, and V represents a protecting group for hydroxy group,
when X represents a R group, W represents a group represented by OZ,
Z represents a group having a structure comprising a solid support and a connecting group, and
when n is an integer of 2 or more, the nucleic acid compound represented by formula (4) may be incorporated by a non-nucleotide linker instead of at least one nucleotide between nucleotides at 5' terminus and 3' terminus of the nucleic acid compound),
the compound containing a phosphate triester bond represents a compound represented by formula (5): 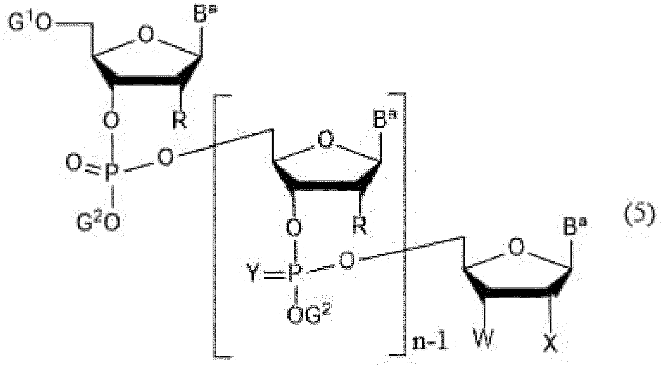 [wherein
G¹, G², B^{a}, R, n, W, X, and Y are the same as defined above, and
as defined in formula (4), a non-nucleotide linker may be incorporated instead of a nucleotide].

3. The process for preparing nucleic acid oligomer according to claim 2, which comprises
a step of elongating a chain strength of a nucleic acid compound represented by formula (5) to any chain length by an amidite method to obtain a nucleic acid represented by formula (5'): 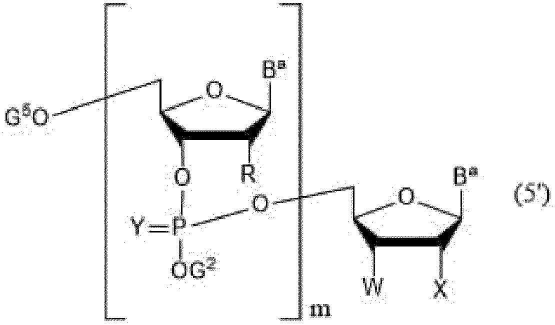 (wherein
G², B^{a}, R, X and W are the same as defied in formula (5),
G⁵ represents a protecting group for hydroxy group, or a hydrogen atom,
m is an integer satisfying m≧n,
Y is the same or different and each independently represents an oxygen atom or a sulfur atom, with the proviso that at least one of Y is an oxygen atom.),
a step of cutting out the compound represented by formula (6): 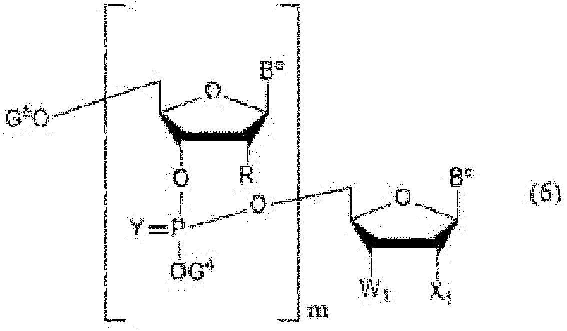 (wherein
G⁵, R and m are the same as defined above,
B^{c} is the same or different and each independently represents a nucleic acid base,
G⁴ represents a hydrogen atom, an alkali metal ion, an ammonium ion, an alkyl ammonium ion, or a hydroxyalkyl ammonium ion,
Y represents independently of each other an oxygen atom or a sulfur atom, and at least one thereof is an oxygen atom, and
X₁ represents a hydroxy group, and W₁ represents a OV group wherein V represents a protecting group for hydroxy group, or
X₁ represents a R group and W₁ represents a hydroxy group)
from the compound represented by formula (5'),
further a step of deprotecting the compound represented by formula (6) to prepare a deprotected nucleic acid oligomer represented by formula (7): 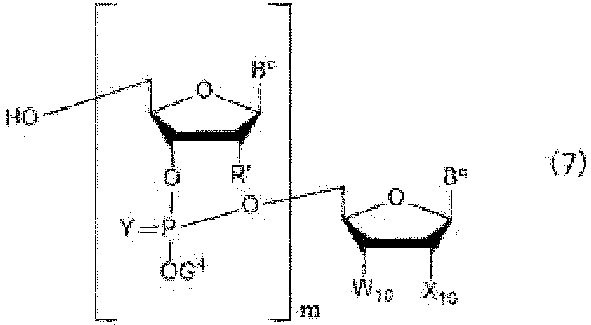 (wherein
m, Y, G⁴, and B^{c} are the same as defined above,
R' is the same or different and each independently represents a hydroxy group, a hydrogen atom, a fluorine atom, a methoxy group, a 2-methoxyethyl group, or a OQ' group,
Q' is the same or different and each independently represents a methylene group attached to a carbon atom at 4'-position of ribose, an ethylene group attached to a carbon atom at 4'-position of ribose, or an ethylidene group attached to a carbon atom at 4'-position of ribose,
X₁₀ and W₁₀ each represents independently of each other a hydroxy group, or
X₁₀ represents an R' group, and W₁₀ represents a hydroxy group).

4. The process according to claim 2 or 3 wherein the non-nucleotide linker is a linker composed by an amino acid backbone.

5. The process according to claim 4 wherein the linker comprising an amino acid backbone is a linker having a structure selected from the following formulae (A14-1), (A14-2) and (A14-3).

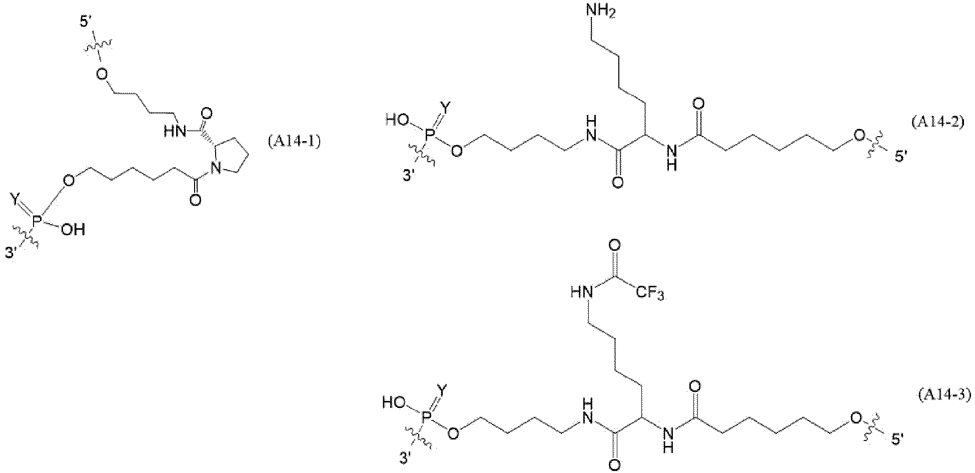

6. The process according to any one of claims 1 to 5 wherein a concentration of iodine in an oxidation solution is 0.005 to 2 M.

7. The process according to any one of claims 1 to 5 wherein a concentration of iodine in an oxidation solution is 0.005 to 0.2 M.

8. The process according to any one of claims 1 to 5 wherein a concentration of iodine in an oxidation solution is 0.007 to 0.1 M.

9. The process according to any one of claims 1 to 5 wherein a concentration of iodine in an oxidation solution is 0.008 to 0.07 M.

10. The process according to any one of claims 1 to 9 wherein the oxidation solution is prepared by mixing iodine, pyridine and water.

11. The process according to claim 10 wherein the oxidation solution is an oxidation solution further containing at least one solvent selected from a group consisting of acetonitrile and tetrahydrofuran.

12. The process according to claim 10 or 11 wherein the oxidation solution is an oxidation solution further containing acetonitrile solvent.

13. The process according to claim 11 wherein a solvent of the oxidation solution is a mixture of solvents which is obtained by mixing pyridine, water, acetonitrile, and tetrahydrofuran in a volume ratio of 1 to 90 : 1 to 50 : 0 to 90 : 0 to 90.

14. The process according to claim 11 or 12 wherein a solvent of the oxidation solution is a mixture of solvents which is obtained by mixing pyridine, water and acetonitrile in a volume ratio of 1 to 90 : 1 to 50 : 0 to 90.

15. The process according to any one of claims 1 to 14 wherein a molar ratio of iodic acid to iodine (mole of iodic acid / mole of iodine) is 25 × 10⁻³ or less.

16. The process according to any one of claims 1 to 14 wherein a molar ratio of iodic acid to iodine (mole of iodic acid / mole of iodine) is 20 × 10⁻³ or less.

17. The process according to any one of claims 1 to 14 wherein a molar ratio of iodic acid to iodine (mole of iodic acid / mole of iodine) is 15 × 10⁻³ or less.

18. The process according to any one of claims 1 to 14 wherein a molar ratio of iodic acid to iodine (mole of iodic acid / mole of iodine) is 10 × 10⁻³ or less.

19. The process according to any one of claims 1 to 14 wherein a molar ratio of iodic acid to iodine (mole of iodic acid / mole of iodine) is 5 × 10⁻³ or less.

20. The process according to any one of claims 1 to 14 wherein a molar ratio of iodic acid to iodine (mole of iodic acid / mole of iodine) is 3 × 10⁻³ or less.

21. The process according to any one of claims 1 to 20 wherein the oxidation solution is an oxidation solution having a duration from a preparation to a use in the oxidation reaction passed over one week or more.

22. The process according to any one of claims 1 to 20 wherein the oxidation solution is an oxidation solution having a duration from a preparation to a use in the oxidation reaction passed over two weeks or more.

23. The process according to any one of claims 1 to 22 wherein the nucleic acid is a ribonucleoside (RNA).

24. The process according to any one of claims 2 to 23 wherein the nucleic acid is ribonucleoside (RNA), and its 2' protecting group is a protecting group represented by formula (12): 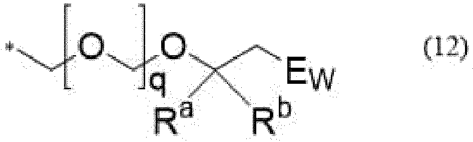 (wherein
q is an integer of 1 to 5,
R^{a} and R^{b} are the same or different and each independently represents a methyl group, an ethyl group, or a hydrogen atom,
a bond marked with symbol * binds to an oxygen atom of OQ group, and
E_{w} represents an electron-attracting group).

25. The process according to claim 24 wherein R^{a} and R^{b} are a hydrogen atom at the same time, and E_{w} represents a cyano group.

26. The process according to any one of claims 1 to 25 wherein the nucleic acid is a ribonucleoside (RNA) comprises 40 or more nucleotides in chain lengths.

27. The process according to any one of claims 1 to 26, which further comprises a step of preparing an oxidation solution described in claim 1.
